(19) Europäisches Patentamt
European Patent Office
Office européen des brevets

(11) Veröffentlichungsnummer: **0 178 521**
**A2**

# (12) EUROPÄISCHE PATENTANMELDUNG

(21) Anmeldenummer: **85112358.8**

(22) Anmeldetag: **30.09.85**

(51) Int. Cl.[4]: **C 07 D 263/58**
A 01 N 43/76

(30) Priorität: **13.10.84 DE 3437638**

(43) Veröffentlichungstag der Anmeldung:
**23.04.86 Patentblatt 86/17**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Mues, Volker, Dr.**
**Gellertweg 13**
**D-5600 Wuppertal 1(DE)**

(72) Erfinder: **Brandes, Wilhelm, Dr.**
**Eichendorffstrasse 3**
**D-5653 Leichlingen(DE)**

(72) Erfinder: **Hänssler, Gerd, Dr.**
**Heymannstrasse 40**
**D-5090 Leverkusen(DE)**

(72) Erfinder: **Reinecke, Paul, Dr.**
**Steinstrasse 8**
**D-5090 Leverkusen 3(DE)**

(54) **2-Benzoxazolyl-iodpropargyl-ether.**

(57) 2-Benzoxazolyl-iodpropargyl-ether der allgemeinen Formel (I)

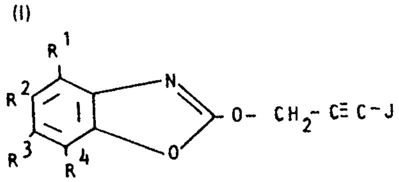

(I)

in welcher

$R^1$, $R^2$, $R^3$ und $R^4$ unabhängig voneinander jeweils für Wasserstoff, Halogen, Cyano, Nitro, Alkyl, Alkoxy, Alkylthio, Alkylcarbonyl, Alkoxycarbonyl, Halogenalkyl, Halogenalkoxy oder Halogenalkylthio stehen,

sowie ihre Verwendung als Schädlingsbekämpfungsmittel.

Die neuen 2-Benzoxazolyl-iodpropargylether der Formel (I) können hergestellt werden, indem man geeignete 2-Benzoxazolylpropargylether mit Jod umsetzt.

EP 0 178 521 A2

BAYER AKTIENGESELLSCHAFT 5090 Leverkusen, Bayerwerk
Konzernverwaltung RP
Patentabteilung Bas/ABc
Ia

## 2-Benzoxazolyl-iodpropargyl-ether

Die Erfindung betrifft neue 2-Benzoxazolyl-iodpropargyl-ether, ein Verfahren zu ihrer Herstellung und ihre Verwendung als Schädlingsbekämpfungsmittel.

Es ist bereits bekannt, daß bestimmte Benztriazolyl-iodpropargyl-ether wie beispielsweise der 4,6-Dichlorbenztriazol-1-yl-iodpropargyl-ether, der 5-Chlorbenztriazol-1-yl-iodpropargyl-ether, der 6-Trifluormethylbenztriazol-1-yl-iodpropargyl-ether, der 6-Methylbenztriazol-1-yl-iodpropargyl-ether oder der 5-Chlor-6-methylbenztriazol-1-yl-iodpropargyl-ether fungizide Eigenschaften besitzen (vgl. z.B. DE-OS 3 105 433).

Die Wirksamkeit dieser vorbekannten Verbindungen ist jedoch insbesondere bei niedrigen Aufwandmengen und Konzentrationen nicht immer in allen Anwendungsbereichen völlig zufriedenstellend.

Es wurden neue 2-Benzoxazolyl-iodpropargyl-ether der allgemeinen Formel (I)

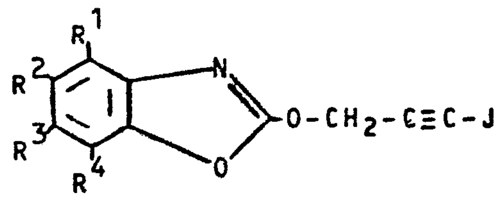

(I)

in welcher

$R^1$, $R^2$, $R^3$ und $R^4$ unabhängig voneinander jeweils für Wasserstoff, Halogen, Cyano, Nitro, Alkyl, Alkoxy, Alkylthio, Alkylcarbonyl, Alkoxycarbonyl, Halogenalkyl, Halogenalkoxy oder Halogenalkylthio stehen,

gefunden.

Man erhält die neuen 2-Benzoxazolyl-iodpropargyl-ether der allgemeinen Formel (I)

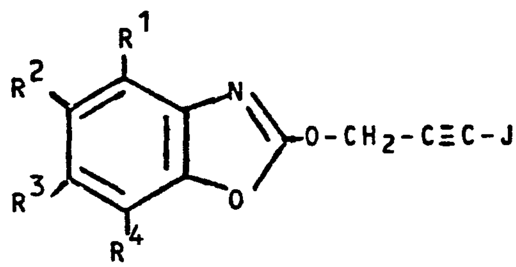

(I)

in welcher

$R^1$, $R^2$, $R^3$ und $R^4$ unabhängig voneinander jeweils für Wasserstoff, Halogen, Cyano, Nitro, Alkyl, Alkoxy, Alkylthio, Alkylcarbonyl, Alkoxycarbonyl, Halogenalkyl, Halogenalkoxy oder Halogenalkylthio stehen,

wenn man Propargylether der Formel (II)

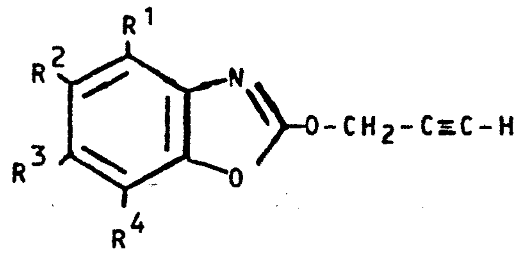

(II)

in welcher

$R^1$, $R^2$, $R^3$ und $R^4$ die oben angegebene Bedeutung haben,

mit Jod, gegebenenfalls in Gegenwart eines Verdünnungsmittels und gegebenenfalls in Gegenwart eines Säureakzeptors umsetzt.

Die neuen Benzoxazolyliodpropargyl-ether der Formel (I) weisen eine mikrobizide Wirkung auf, vor allem eine starke fungizide Wirkung.

Überraschenderweise zeigen die erfindungsgemäßen 2-Benzoxazolyl-iodpropargyl-ether der Formel (I) eine erhebliche bessere fungizide Wirksamkeit als die aus dem Stand der Technik bekannten Benztriazolyl-iodpropargyl-ether, wie beispielsweise der 4,6-Dichlorbenztriazol-1-yl-iodpropargylether, der 5-Chlor-benztriazol-1-yl-iodpropargyl-ether, der 6-Trifluormethylbenztriazol-1-yl-iodpropargyl-ether, der 6-Methylbenztriazol-1-yl-iodpropargyl-ether oder der 5-Chlor-6-methyl-benztriazol-1-yl-iodpropargyl-ether, welches chemisch und wirkungsmäßig naheliegende Verbindungen sind.

Die erfindungsgemäßen 2-Benzoxazolyl-iodpropargylether sind durch die Formel (I) allgemein definiert. Bevorzugt sind Verbindungen der Formel (I), bei welchen

$R^1$, $R^2$, $R^3$ und $R^4$ unabhängig voneinander jeweils für Wasserstoff, Halogen, Cyano, Nitro, für jeweils geradkettiges oder verzweigtes Alkyl, Alkoxy, Alkylthio, Alkylcarbonyl, Alkoxycarbonyl, Halogenalkyl, Halogenalkoxy oder Halogenalkylthio mit jeweils 1 bis 4 Kohlenstoffatomen je Alkyl- oder Alkoxyteil und gegebenenfalls 1 bis 9 gleichen oder verschiedenen Halogenatomen stehen.

Besonders bevorzugt sind Verbindungen der Formel (I), bei welchen

$R^1$, $R^2$, $R^3$ und $R^4$ unabhängig voneinander jeweils für Wasserstoff, Fluor, Chlor, Brom, Jod, Cyano, Nitro, Methyl, Ethyl, n- oder i-Propyl, Methoxy, Ethoxy, n- oder i-Propoxy, Methylthio, Ethylthio, Trifluormethyl, Trichlormethyl, Trifluormethoxy, Trifluormethylthio, Acetyl, Methoxycarbonyl oder Ethoxycarbonyl stehen.

Im einzelnen seien außer den bei den Herstellungsbeispielen genannten Verbindungen die folgenden 2-Benzoxazolyl-

iodpropargyl-ether der allgemeinen Formel (I)

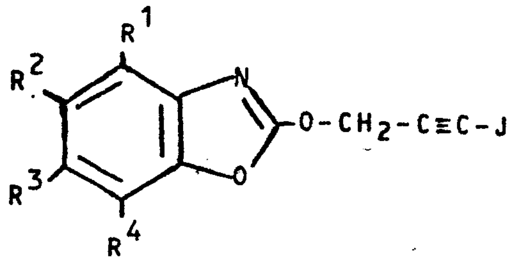

(I)

genannt:

| $R^1$ | $R^2$ | $R^3$ | $R^4$ |
|---|---|---|---|
| Br | H | H | H |
| H | Br | H | H |
| H | H | H | Br |
| Cl | H | H | H |
| H | H | Cl | H |
| Br | Br | H | H |
| Br | H | Br | H |
| Br | H | H | Br |
| H | H | $CF_3$ | H |
| H | H | CN | H |
| H | $NO_2$ | H | H |
| H | H | $CCl_3$ | H |
| $CH_3$ | $CH_3$ | H | H |
| H | Cl | $CH_3$ | H |
| Cl | $CH_3$ | Cl | H |
| H | CN | H | H |
| H | $CH_3$ | $CH_3$ | H |
| H | H | $NO_2$ | H |

Verwendet man beispielsweise 2-Propargyloxybenzoxazol und Jod als Ausgangsstoffe und Natriumhydroxid als Säureakzeptor, so läßt sich der Reaktionsablauf des erfindungsgemäßen Verfahrens durch das folgende Formelschema darstellen:

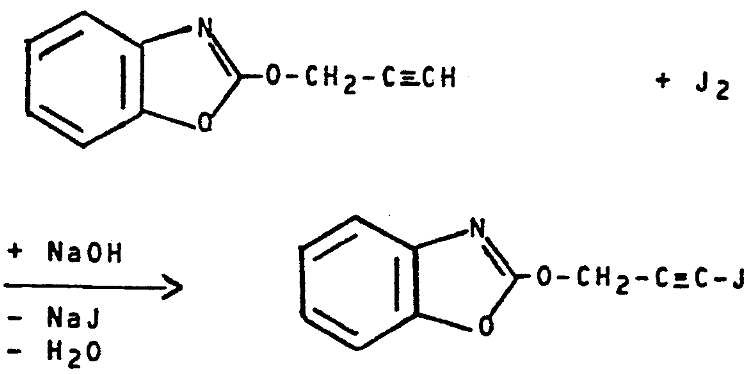


Die zur Durchführung des erfindungsgemäßen Verfahrens als Ausgangsstoffe benötigten Propargylether sind durch die Formel (II) allgemein definiert.

Bevorzugt sind Verbindungen der Formel (II), bei welchen $R^1$, $R^2$, $R^3$ und $R^4$ für diejenigen Substituenten stehen, die bereits bei der Beschreibung der erfindungsgemäßen Stoffe der Formel (I) als bevorzugt für diese Reste genannt wurden.

Die Propargylether der Formel (II) sind bekannt (vgl. z.B. JP-A 54 020 137, DE-OS 3 026 957, EP 17 810) oder lassen sich nach prinzipiell bekannten Verfahren in analoger Weise herstellen.

Für die erfindungsgemäße Umsetzung kommen als Verdünnungsmittel praktisch alle organischen Solventien in Frage. Hierzu gehören insbesondere Alkohole, wie Methanol, Ethanol, n- und iso-Propanol, n-, iso-, sek.- und tert.-Butanol; Ether wie Diethylether, Dibutylether, Tetrahydrofuran und Dioxan, Ketone wie Aceton, Methylethylketon, Methylisopropylketon und Methylisobutylketon; Nitrile wie Acetonitril und Propionsäurenitril, sowie die hochpolaren Lösungsmittel Dimethylformamid, Dimethylsulfoxid, Sulfolan und Hexamethylphosphorsäuretriamid.

Als Säureakzeptoren können praktisch alle üblicherweise verwendbaren Säurebindemittel eingesetzt werden; hierzu gehören insbesondere Alkali- und Erdalkalihydroxide bzw. -oxide, z.B. Natriumhydroxid und Calcium-hydroxid; Alkali- und Erdalkalicarbonate wie Natrium-, Kalium- und Calciumcarbonat; Alkalialkoholate wie Natrium-methylat, -ethylat und -tert.-butylat; ferner aliphatische, aromatische oder heterocyclische Amine wie Triethylamin, Pyridin, Diazabicyclononen (DBN) und Diazabicycloundecen (DBU).

Die Reaktionstemperatur kann innerhalb eines größeren Bereichs variiert werden. Im allgemeinen arbeitet man zwischen -50°C und +150°C, vorzugsweise bei -20°C bis +100°C. Das erfindungsgemäße Verfahren wird im allgemeinen bei Normaldruck durchgeführt.

Bei der Durchführung des erfindungsgemäßen Verfahrens setzt man auf 1 Mol der Verbindungen der Formel (II) 1,0 bis 1,5 Mol Jod und gegebenenfalls 1 bis 2 Mol Säurebinder ein.

Zur Durchführung des erfindungsgemäßen Verfahrens werden vorzugsweise die Ausgangsstoffe der Formel (II) und das Säurebindemittel in einem der oben genannten Verdünnungsmittel vorgelegt, mit Jod versetzt und bis zum Ende der Umsetzung gerührt. Die Produkte der Formel (I) werden durch Absaugen isoliert. Zur Charakterisierung dient der Schmelzpunkt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet, vor allem als Fungizide.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft, aber nicht begrenzend, seien einige Erreger von pilzlichen Erkrankungen, die unter die oben aufgezählten Oberbegriffe fallen, genannt:

| | |
|---|---|
| Botrytis-Arten, | wie beispielsweise Botrytis cinerea; |
| Plasmopara-Arten, | wie beispielsweise Plasmopara viticola; |
| Uromyces-Arten, | wie beispielsweise Uromyces appendiculatus; |
| Sphaerotheca-Arten, | wie beispielsweise Sphaerotheca fuliginea; |
| Venturia-Arten, | wie beispielsweise Venturia inaequalis; |
| Podosphaera-Arten, | wie beispielsweise Podosphaera leucotricha; |
| Phytophthora-Arten, | wie beispielsweise Phytophthora infestans; |

Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Utilago nuda oder Ustilago avenae;
Septoria-Arten, wie beispielsweise Septoria nodorum;
Tilletia-Arten, wie beispielsweise Tilletia caries;
Pyricularia-Arten, wie beispielsweise Pyricularia oryzae;
Pellicularia-Arten, wie beispielsweise Pellicularia sasakii;
Erwinia-Arten, wie beispielsweise Erwinia amylovora;
Pyrenophora-Arten, wie beispielsweise Pyrenophora teres;
(Konidienform: Drechslera, Syn: Helminthosporium);
Leptosphaeria-Arten, wie beispielsweise Leptosphaeria nodorum;
Cochliobolus-Arten, wie beispielsweise Cochliobolus sativus (Konidienform: Drechslera, Syn: Helminthosporium) und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen den Erreger des Getreidemehltaus (Erysiphe graminis), zur Bekämpfung von Obst- und Gemüsekrankheiten, wie beispielsweise gegen den Erreger des Apfelschorfs (Venturia inaequalis) oder gegen den Erreger der Tomatenfäule (Phytophthora infestans), oder zur Bekämpfung von Reiskrankheiten, wie beispielsweise

gegen den Erreger der Reisfleckenkrankheit (Pyricularia oryzae) einsetzen.

In geeigneten Aufwandmengen besitzen die erfindungsgemäßen Wirkstoffe neben der fungiziden auch eine akarizide Wirksamkeit.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol

oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige,

körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B.

durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1:

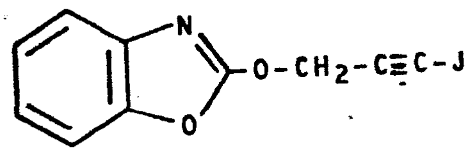

19 g (0,11 Mol) 2-Propargyloxy-1,3-benzoxazol in 100 ml Methanol werden unter Rühren portionsweise mit 12 g (0,12 Mol) 40-prozentiger wäßriger Natronlauge und 27,9 g (0,11 Mol) Jod versetzt und nach beendeter Zugabe für 2 Stunden bei 40°C und weitere 15 Stunden bei Raumtemperatur nachgerührt. Der ausgefallene Niederschlag wird abgesaugt und getrocknet.

Man erhält 18,2 g (61 % der Theorie) an 2-Jodpropargyloxy-1,3-benzoxazol vom Schmelzpunkt 147°C.

Herstellung der Ausgangsverbindung

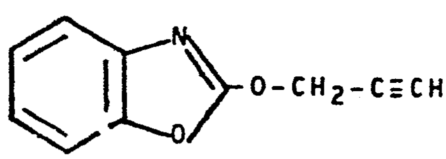

Zu 435 ml (422,6 g; 7,5 Mol) Propargylalkohol gibt man bei 20°C unter Rühren zunächst 62 g (1,1 Mol) gepulvertes Kaliumhydroxid und danach bei 30°C 153 g (1,0 Mol) 2-Chlorbenzoxazol und rührt nach beendeter Zugabe weitere

2 Stunden bei Raumtemperatur, gießt die Reaktionsmischung in Wasser, extrahiert zweimal mit Toluol, wäscht die vereinigten organischen Phasen dreimal mit Wasser, trocknet über Natriumsulfat und entfernt das Lösungsmittel bei 45°C Badtemperatur im Vakuum.

Man erhält 164,4 g (95 % der Theorie) an 2-Propargyloxy-1,3-benzoxazol als Öl.

In entsprechender Weise und gemäß den allgemeinen Herstellungsangaben erhält man die folgenden 2-Benzoxazolyliodpropargyl-ether der allgemeinen Formel (I):

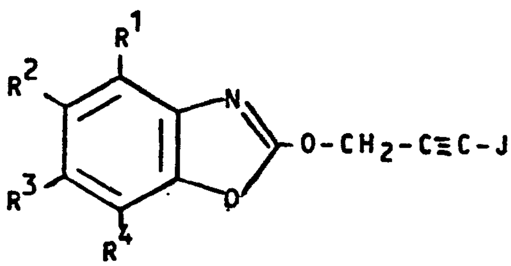

(I)

| Bsp. Nr. | $R^1$ | $R^2$ | $R^3$ | $R^4$ | Schmelzpunkt (°C) |
|---|---|---|---|---|---|
| 2 | H | $CH_3$ | H | H | 117 |
| 3 | H | Cl | H | H | 81 |

Anwendungsbeispiele

In den folgenden Anwendungsbeispielen werden die nachstehend aufgeführten Verbindungen als Vergleichssubstanzen eingesetzt:

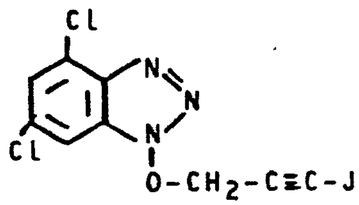

(A)

4,5-Dichlorbenztriazol-1-yl-iodpropargyl-ether

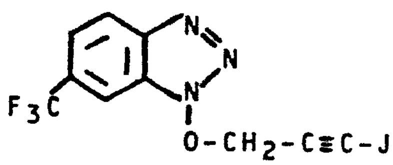

(B)

6-Trifluormethyl-bentriazol-1-yl-iodpropargyl-ether

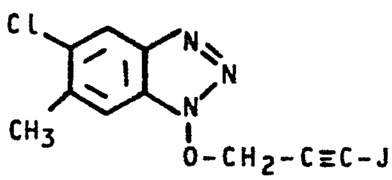

(C)

5-Chlor-6-methyl-benztriazol-1-yl-iodpropargyl-ether

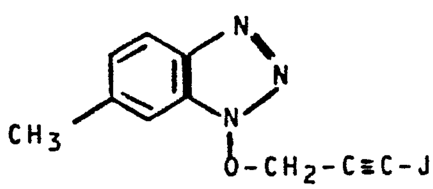

(D)

6-Methylbenztriazol-1-yl-iodpropargyl-ether

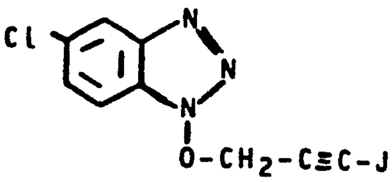

(E)

5-Chlorbenztriazol-1-yl-iodpropargyl-ether

(alle bekannt aus DE-OS 3 105 433).

Beispiel A

Phytophthora-Test (Tomate) /protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100 % relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1 und 2.

Beispiel B

Venturia-Test (Apfel) / protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1 und 2.

0178521

Beispiel C

Pyricularia-Test (Reis) / protektiv

Lösungsmittel: 12,5 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigt in diesem Test z.B. die Verbindung gemäß dem Herstellungsbeispiel: 1.

Beispiel D

Erysiphe-Test (Gerste) / protektiv

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 1 und 2.

Patentansprüche

1. 2-Benzoxazolyl-iodpropargyl-ether der Formel (I)

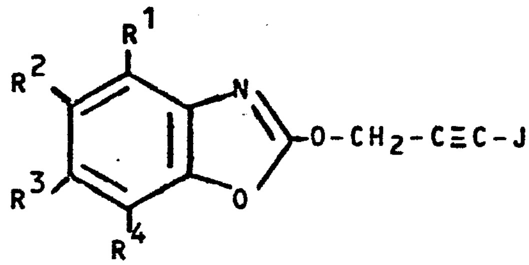

(I)

in welcher

$R^1$, $R^2$, $R^3$ und $R^4$ unabhängig voneinander jeweils für Wasserstoff, Halogen, Cyano, Nitro, Alkyl, Alkoxy, Alkylthio, Alkylcarbonyl, Alkoxycarbonyl, Halogenalkyl, Halogenalkoxy oder Halogenalkylthio stehen.

2. 2-Benzoxazolyl-iodpropargyl-ether der Formel (I) gemäß Anspruch 1, in welcher

$R^1$, $R^2$, $R^3$ und $R^4$ unabhängig voneinander jeweils für Wasserstoff, Halogen, Cyano, Nitro oder für jeweils geradkettiges oder verzweigtes Alkyl, Alkoxy, Alkylthio, Alkylcarbonyl, Alkoxycarbonyl, Halogenalkyl, Halogenalkoxy oder Halogenalkylthio mit jeweils 1 bis 4 Kohlenstoffatomen je Alkyl- oder Alkoxyteil und gegebenenfalls 1 bis 9 gleichen oder verschiedenen Halogenatomen stehen.

3. 2-Benzoxazolyl-iodpropargyl-ether der Formel (I) gemäß Anspruch 1, in welcher

$R^1$, $R^2$, $R^3$ und $R^4$ unabhängig voneinander jeweils für Wasserstoff, Fluor, Chlor, Brom, Jod, Cyano, Nitro, Methyl, Ethyl, n- oder i-Propyl, Methoxy, Ethoxy, n- oder i-Propoxy, Methylthio, Ethylthio, Trifluormethyl, Trichlormethyl, Trifluormethoxy, Trifluormethylthio, Acetyl, Methoxycarbonyl oder Ethoxycarbonyl stehen.

4. Verfahren zur Herstellung von 2-Benzoxazolyl-iodpropargyl-ethern der Formel (I)

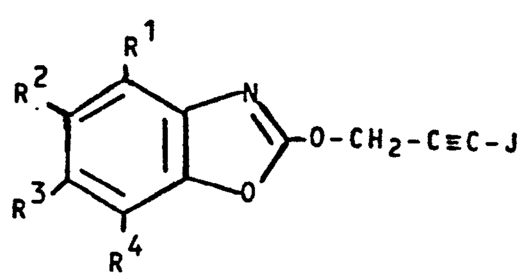

(I)

in welcher

$R^1$, $R^2$, $R^3$ und $R^4$ unabhängig voneinander jeweils für Wasserstoff, Halogen, Cyano, Nitro, Alkyl, Alkoxy, Alkylthio, Alkylcarbonyl, Alkoxycarbonyl,

Halogenalkyl, Halogenalkoxy oder Halogenalkylthio stehen,

dadurch gekennzeichnet, daß man Propargylether der Formel (II)

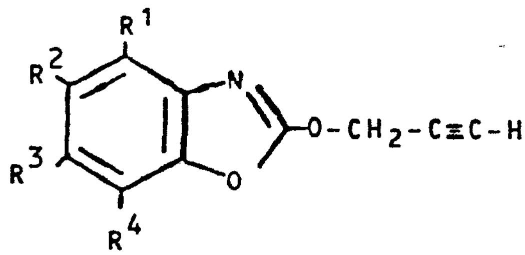

(II)

in welcher

$R^1$, $R^2$, $R^3$ und $R^4$ die oben angegebene Bedeutung haben,

mit Jod, gegebenenfalls in Gegenwart eines Verdünnungsmittels und gegebenenfalls in Gegenwart eines Säureakzeptors umsetzt.

5. Schädlingsbekämpfungsmittel, gekennzeichnet durch einen Gehalt an mindestens einem 2-Benzoxazolyl-iodpropargyl-ether der Formel (I).

6. Verfahren zur Bekämpfung von Schädlingen, dadurch gekennzeichnet, daß man 2-Benzoxazolyl-iodpropargylether der Formel (I) auf Schädlinge und/oder ihren Lebensraum einwirken läßt.

7. Verwendung von 2-Benzoxazolyl-iodpropargyl-ethern der Formel (I) zur Bekämpfung von Schädlingen.

8. Verfahren zur Herstellung von Schädlingsbekämpfungsmitteln, dadurch gekennzeichnet, daß man 2-Benzoxazolyl-iodpropargyl-ether der Formel (I) mit Streckmitteln und/oder oberflächenaktiven Mitteln vermischt.

9. Fungizide Mittel, gekennzeichnet durch einen Gehalt an mindestens einem 2-Benzoxazolyl-iodpropargyl-ether der Formel (I).

10. Verwendung von 2-Benzoxazolyl-iodpropargyl-ethern der Formel (I) gemäß Anspruch 7 zur Bekämpfung von Pilzen.